# EUROPÄISCHE PATENTSCHRIFT

(11) **EP 0 555 697 B1**
(45) Veröffentlichungstag und Bekanntmachung des Hinweises auf die Patenterteilung: **19.06.1996**
(21) Anmeldenummer: 93101288.4
(22) Anmeldetag: 28.01.1993
(51) Int. Cl.: C07C 68/00, C07C 69/96

(54) **Verfahren zur Herstellung von Dialkylcarbonaten**
Process for the preparation of dialkyle carbonates
Procédé de préparation de carbonates de dialkyle

(30) Priorität: 10.02.1992 DE 4203796
(43) Veröffentlichungstag der Anmeldung: 18.08.1993
(73) Patentinhaber: BAYER AG, 51368 Leverkusen (DE)
(72) Erfinder: Rechner, Johann, Dr., W-4150 Krefeld (DE); Klausener, Alexander, Dr., W-500 Koeln 1 (DE); Buysch, Hans-Josef, Dr., W-4150 Krefeld (DE); Wagner, Paul, Dr., W-4000 Düsseldorf 13 (DE)

(56) Entgegenhaltungen:
- DE-A- 2 743 690
- DE-A- 3 926 709

## Beschreibung

Die vorliegende Erfindung betrifft ein Verfahren zur kontinuierlichen Herstellung von Dialkylcarbonaten durch Umsetzung des entsprechenden Alkanols mit Sauerstoff und Kohlenmonoxid in Gegenwart eines im Reaktionsmedium suspendierten oder gelösten kupferhaltigen Katalysators, bei erhöhtem Druck und erhöhter Temperatur, bei dem der Katalysator durch Sedimentation und das Reaktionswasser im besonders wichtigen Falle des Methanols durch einfache Destillation aus dem Reaktionsmedium entfernt werden.

In den vergangenen Jahren wurden eine Reihe von Verfahren zur Herstellung von Dialkylcarbonaten durch die katalytische Umsetzung der Edukte Alkanol, Kohlenmonoxid und Sauerstoff entwickelt.

In DE-A 2 110 194 wurden als geeignete Katalysatoren Metallkomplexe der Gruppen IB, IIB und VIIIB des Periodensystems genannt, insbesondere solche der Metalle Cu, Ag, Au, Zn, Cd, Hg, Fe, Co und Ni, die bei Redoxreaktionen in zwei verschiedenen Oxidationsstufen existieren können.

Dieses Verfahren liefert mit Cu₂Cl₂ gute Ausbeuten, hat aber den Nachteil, daß die Abtrennung der sehr teuren Komplexliganden und des gelösten komplexierten Katalysators von der Reaktionslösung aufwendig ist.

In DE-C 2 743 690 werden statt der Kupferkomplexverbindungen einfache einwertige Salze des Kupfers als Katalysatoren verwendet. Diese Verfahrensvariante liefert zwar gute Ausbeuten an Dialkylcarbonaten, aber auch hier bereitet die Aufarbeitung der Reaktionslösung große Probleme, da der teilweise gelöste Katalysator von der Reaktionslösung abgetrennt werden muß. Nach der Lehre des Patents erfolgt dies durch Abfiltrieren des suspendierten Anteils und Rektifizierung oder Kristallisation des gelösten Katalysators. Da die katalysatorhaltigen Reaktionslösungen den Katalysator in weitere Anlagenteile tragen, ist für die Aufarbeitung der Reaktionslösung und des Katalysators ein hoher apparativer Aufwand erforderlich. Aufgrund der korrosiven Eigenschaften müssen alle Apparate (Kessel, Rohrleitungen, Destillations-, Kristallisations- und Filtrationsapparate), die mit dem Katalysator in Kontakt kommen, aus korrosionsbeständigem Material bestehen. Hierdurch verliert das Verfahren an Attraktivität.

Die gleichen Aufarbeitungsprobleme fuhren dazu, daß die Verwendung von Synthesegas statt CO, wie in DE-C 3 045 767 beschrieben, wirtschaftlich unattraktiv bleiben muß. Die durch den kupferhaltigen Katalysator verursachten Korrosionsprobleme bei der Aufarbeitung der Reaktionslösung lassen Verfahren, die weitere Zusätze zum Katalysator enthalten (z.B. EP-A 217 651, EP-B 90 977, US-A 4 370 275) unwirtschaftlich werden.

Eine verfahrenstechnische Alternative zur Abtrennung des Katalysators lehrt DE-A 3 926 709; hierbei verbleibt der kupferhaltige Katalysator im Reaktor. Das während der Reaktion gebildete Dialkylcarbonat wird zusammen mit dem Reaktionswasser und Alkanol durch das Reaktionsgas aus der Reaktionsmischung gestrippt. Dieser Effekt wird im allgemeinen dadurch erzielt, daß ein Gasstrom von 20 bis 30 NL CO/O₂-Gasgemisch pro g im Reaktor als Kupferkatalysator vorhanden Kupfers durch die Reaktionsmischung geleitet wird. Nachteilig bei diesem Verfahren sind die sehr großen Gasmengen, die im Kreislauf gehalten werden müssen und die hierdurch verursachten hohen Energiekosten, sowie aufgrund der großen Gasmengen die Probleme bei der Gasdispergierung. Bei dieser Fahrweise müssen zusätzlich die Temperatur und der Druck des Reaktors sehr genau geregelt werden, um den Flüssigkeitsstand im Reaktor halten zu können, da bereits geringe Schwankungen der Reaktortemperatur oder des Druckes zu deutlich veränderten Austragsmengen führen. Außerdem wird dabei eine relativ große Menge Wasser im Reaktor angesammelt, die die Selektivität der Reaktion erniedrigt.

Die Reaktion wird in den beschriebenen Verfahren in Autoklaven als Druckgefäß durchgeführt. Diese teuren Reaktoren haben den Nachteil, daß die Durchmischung des Reaktionsmediums mit Hilfe von Rührorganen und Einbauten erfolgt, die einem hohen Verschleiß unterliegen. Zusätzlich stellt speziell die Rührereinheit eine potentielle Undichtigkeitsquelle dar.

In EP 460 732 A1 und EP 460 735 A2 wird ein Verfahren beschrieben, bei dem analog zur DE-A 3 926 709 das während der Reaktion gebildete Dialkylcarbonat zusammen mit Alkanol und Reaktionswasser durch das Reaktionsgas aus dem Reaktor entfernt wird. In EP 460 735 A2 wird ein speziell dimensionierter Schlaufenreaktor mit externem Stoffkreislauf beschrieben, der die oben genannten Nachteile nicht haben soll. Dieser Reaktor bildet jedoch leicht Totzonen aus, insbesondere im unteren Krümmerbereich, besitzt ein großes Bauvolumen, einen zusätzlichen Wärmetauscher und weist Dichtigkeitsprobleme auf wegen der vielen Flanschverbindungen. Das in den beiden Patentanmeldungen beschriebene Verfahren arbeitet mit sehr hohen Wassergehalten im Reaktor, was nach U. Romano, R. Tesel, M.M. Mauri und P. Rebora (Ind. Eng. Chem. Prod. Res. Dev., (1980), 19, 400) zu einem CO-Selektivitätsverlust und einer erhöhten CO₂-Produktion führt. In EP-A 460 735 wird z.B. im Beispiel 1 lediglich eine CO-Selektivität für DMC von 77 % erzielt; die restliche Selektivität entfällt auf die CO₂-Bildung. In EP-A 460 732 liegen die erzielten DMC-Selektivitäten bezüglich Methanol lediglich bei 95-97 %, wie aus den Beispielen hervorgeht. Diese Nachteile und die bereits bei DE-A 3 926 709 aufgeführten Nachteile führen dazu, daß diese Verfahrensvariante unwirtschaftlich ist.

Ein weiteres Verfahrensproblem ist die Abtrennung des Reaktionswassers, beispielsweise vom Methanol und Dimethylcarbonat. Nach DE-OS 2 450 856 gestaltet sich die Abtrennung des Dimethylcarbonates vom Reaktionswasser und Methanol mittels einer einfachen Rektifikation kompliziert, da sich verschiedene Azeotrope zwischen DMC, MeOH und Wasser bilden. Die Patentanmeldung lehrt eine Trennung durch Verwendung einer Extraktivdestillation unter Verwendung von Wasser als Lösungsmittel. Das Verfahren ist jedoch unwirtschaftlich, da erhebliche Wassermengen für die Trennung erforderlich sind (9,5 g Wasser auf 1 g Reaktionslösung).

Es bestand nun die Aufgabe, ein Verfahren zur Herstellung von Dialkylcarbonaten aus Alkanol, Kohlenmonoxid und Sauerstoff zu finden, das eine wirtschaftliche Herstellung von Dialkylcarbonat bei insbesondere hohen Raum-Zeit-Ausbeuten ermöglicht und eine einfache kontinuierliche Abtrennung des im Kreislauf geführten Katalysators und des Reaktionswassers von der Flüssigphase erlaubt.

Es wurde nun gefunden, daß man die Reaktion in einem Schlaufenreaktor mit internem Stoffkreislauf durchführen und den Katalysator aus der abgetrennten Reaktionslösung durch einfache Sedimentation abtrennen kann, wenn man die Abtrennung im Temperaturbereich von 20 bis 200°C und einem Sauerstoffpartialdruck in der Gasphase von höchstens 5 Vol.-% durchführt. Weiter wurde gefunden, daß man aus dem flüssigen Reaktionsprodukt im Falle von Methanol als Alkanol das Reaktionswasser durch einfache Reaktifikation entfernen kann, wobei das Wasser als Sumpfprodukt und das Azeotrop DMC/Methanol und überschüssiges Methanol als Kopfprodukt anfallen.

Die vorliegende Erfindung betrifft demnach ein Verfahren zur Herstellung von Dialkylcarbonaten der Formel

RO-CO-OR (I),

in der
- R: für C₁-C₁₀-Alkyl, bevorzugt für C₁-C₂-Alkyl, besonders bevorzugt für CH₃ steht,
durch Umsetzung des zugrunde liegenden Alkanols ROH mit Sauerstoff und Kohlenmonoxid in Gegenwart eines Kupferkatalysators bei erhöhter Temperatur und erhöhtem Druck, das dadurch gekennzeichnet ist, daß der Katalysator in unlöslicher Form durch mechanische Abtrennung bei 20 bis 200°C, bevorzugt bei 40 bis 150°C, besonders bevorzugt bei 40 bis 120°C und einem Sauerstoff-Partialdruck über dem Reaktionsgemisch von höchstens 5 Vol.-%, bevorzugt höchstens 1 Vol.-%, besonders bevorzugt höchstens 0,5 Vol.-% abgetrennt wird und das vom Katalysator befreite Reaktionsgemisch durch Rektifikation aufgearbeitet wird.

Das erfindungsgemäße Verfahren kann diskontinuierlich oder kontinuierlich, bevorzugt kontinuierlich durchgeführt werden. Das Alkanol wird im Reaktor in Gegenwart des Katalysators mit Sauerstoff und Kohlenmonoxid umgesetzt. Bei dieser Umsetzung kann auch Inertgas anwesend sein. Als Inertgas kommen die neben O₂ vorhandenen Bestandteile der Luft und Edelgase in Betracht, ferner CO₂ und geringe Mengen Kohlenwasserstoffe, die beispielsweise in einem CO aus Reformern enthalten sein können. Das bei der Umsetzung gebildete Dialkylcarbonat und das Reaktionswasser werden zusammen mit überschüssigem Alkanol und dem Katalysator aus dem Reaktor entnommen. Wenn die Reaktionsmischung bei 20-200°C und einem O₂-Partialdruck über der Reaktionsmischung von höchstens 5 Vol.-% gehalten wird, bildet sich eine unlösliche und rasch sedimentierende Form des Katalysators. Die Abtrennung des Katalysators in dieser unlöslichen Form von der Reaktionslösung erfolgt durch absatzweise oder kontinuierliche Sedimentation des Katalysators. Als Sedimentation im Sinne der Erfindung ist die Abtrennung des Katalysators durch die Einwirkung des Erdschwerefeldes und/oder eines Zentrifugalkraftfeldes zu verstehen.

Alternativ kann die Abtrennung der Reaktionslösung vom sedimentierten Katalysator bei diskontinuierlicher Fahrweise auch im Reaktor, beispielsweise durch Abdekantieren oder Abhebern, vorgenommen werden; anschließend wird dem Katalysator im Reaktor wieder die entsprechende Menge Alkanol zugeführt und die Reaktion erneut durchgeführt. Bei dieser Art der Fahrweise verbleibt der Katalysator im Reaktor, dies hat den Vorteil, daß nachgeschaltete Apparate nicht korrosionsfest ausgelegt werden müssen. Durch Parallelschaltung zweier oder mehrerer diskontinuierlicher Reaktoren ist die Aufarbeitung der katalysatorfreien Reaktionslösung von der diskontinuierlichen Fahrweise nicht betroffen und kann weiterhin in der bevorzugten kontinuierlichen Ausführungsform erfolgen.

In der bevorzugten kontinuierlichen Ausführungsform wird der zu recyclisierende Katalysator als Suspension zusammen mit frischem und recyclisiertem Alkanol dem Reaktor zugeführt und gleichzeitig die entsprechende Menge Reaktionslösung und Katalysator dem Reaktor entnommen. Als Reaktor wird in der bevorzugten Ausführungsform ein Schlaufenreaktor mit internem Stoffkreislauf verwendet, womit eine gute Durchmischung der Suspension mit der Gasphase ohne Rührer bei gleichzeitig geringem Reaktionsvolumen erzielt wird.

Diese Art der Fahrweise hat den Vorteil, daß der Reaktor kontinuierlich betrieben werden kann und somit eine höhere Raum-Zeit-Ausbeute möglich ist. Da durch die vollständige Katalysatorabtrennung nur wenige Apparate der Korrosion ausgesetzt werden, reduzieren sich die Investitionskosten aufgrund der geringen Zahl korrosionsfest auszuführender Apparate beträchtlich gegenüber dem oben angeführten Stand der Technik.

Die so erhaltene katalysatorfreie, klare und wasserhelle Reaktionslösung wird in einer einfachen Rektifikation vom Reaktionswasser befreit, wobei man beispielsweise im Falle von Methanol als Kopfprodukt das Azeotrop aus Methanol/DMC sowie überschüssiges Methanol und als Sumpfprodukt das Reaktionswasser erhält; ein niedrigsiedendes ternäres Azeotrop aus Dialkylcarbonat, Wasser und Methanol wurde hingegen nicht beobachtet.

Im erfindungsgemäßen Verfahren werden als Katalysatoren Kupferverbindungen auf der Basis von Kupfer(I)- und/oder Kupfer(II)-salzen verwendet. Hierbei handelt es sich um anorganische bzw. organische Cu-Salze, die auch als Alkoxysalze mit C₁-C₄-Alkoxy vorliegen können. Da es sich bei der Reaktion um eine Redoxreaktion handelt, liegen während der Umsetzung beide Kupferionenspezies vor. Als Kupferkatalysatoren werden vorzugsweise Kupfer(I)-halogenide, Kupfer(I)-acetylacetonat, Kupfer(I)-sulfat und/oder Kupfer(II)-alkoxyhalogenide, Kupfer(II)-alkoxysulfat, Kupfer(II)-alkoxyacetylacetonat eingesetzt, besonders bevorzugt werden Kupfer(I)-chlorid und/oder Kupfer(II)-methoxychlorid eingesetzt.

Das flüssige Reaktionsmedium besteht zum größten Teil aus dem umzusetzenden C₁-C₁₀-Alkanol, bevorzugt C₁-C₂-Alkanol und besonders bevorzugt Methanol. Im allgemeinen beträgt das bezüglich des Alkanolgehaltes auf 1 normierte Molverhältnis von Alkanol:Dialkylcarbonat:Kupfer (Kupfer aus dem im Reaktionsmischung suspendierten und/oder gelösten Katalysator) im Reaktionsgemisch bei Reaktionsende beziehungsweise beim Verlassen des Reaktors im kontinuierlichen Betrieb 1;(0,005-1):(0,0001-5), vorteilhaft 1:(0,02-0,5):(0,0005-1) und besonders bevorzugt 1:(0,04-0,3):(0,001-0,2).

Die Umsetzung der Reaktionsgase mit dem Alkanol kann bei einer Temperatur von 60 bis 200°C, bevorzugt von 80 bis 140°C und besonders bevorzugt von 100 bis 130°C betrieben werden; dabei kann bei Normaldruck oder unter erhöhtem Druck gearbeitet werdend. Im allgemeinen wird die Reaktion bei Drucken von 1 bis 60 bar, bevorzugt bei 10 bis 40 und besonders bevorzugt bei 15 bis 35 bar durchgeführt. Der Druck wird zweckmäßigerweise durch Aufpressen der Reaktionsgase erzeugt.

Der dem Reaktor zugeführte Gasstrom kann in weiten Grenzen variiert werden, es wird jedoch zweckmäßigerweise ein Gesamtgasstrom, bestehend aus CO, Sauerstoff und eventuell einem Inertgas (wie z.B. N₂, CO₂ usw.), bezogen auf das in der Reaktionslösung vorliegende Kupfer des Katalysators, von 0,2 bis 2000 NL/h pro g Cu und besonders bevorzugt von 0,8 bis 1500 NL/h pro g Cu eingestellt.

Die Zusammensetzung der Reaktionsgase Kohlenmonoxid und Sauerstoff kann in weiten Konzentrationsgrenzen variiert werden, es wird jedoch zweckmäßigerweise ein CO:O₂-Molverhältnis (normiert auf CO) von 1:(0,005-1,0) und bevorzugt von 1:(0,01-0,5) eingestellt. Der Sauerstoffpartialdruck ist bei diesen Molverhältnissen groß genug, um hohe Raum-Zeit-Ausbeuten erreichen zu können und gleichzeitig keine explosionsfähigen Kohlenmonoxid/Sauerstoff-Gasgemische bilden zu können. Die Reaktionsgase unterliegen keinen besonderen Reinheitsanforderungen, so kann Synthesegas als CO-Quelle und Luft als O₂-Träger dienen, es ist jedoch darauf zu achten, daß keine Katalysatorgifte wie z.B. Schwefel oder dessen Verbindungen eingetragen werden.

Die Umsetzung des katalysatorhaltigen Alkanols bei Reaktionsbedingungen mit den Reaktionsgasen wird zweckmäßigerweise bei einer möglichst niedrigen Konzentration des zwangsläufig anfallenden Reaktionswassers in der Reaktionsmischung durchgeführt, um Nebenreaktionen wie die Bildung von Kohlendioxid und die gleichzeitige Desaktivierung des Kupferkatalysators, zu vermeiden. Die Reaktionswasserkonzentration beträgt im allgemeinen maximal 8 Gew.-%, vorteilhaft maximal 6 Gew.-%, bezogen auf die flüssige Phase.

Die Umsetzung wird bis zu einem gewünschten und einstellbaren Wert, bezogen auf das eingesetzte Alkanol, von kleiner 35 % und bevorzugt von kleiner 25 % getrieben und die Reaktionslösung im Reaktor oder bevorzugt in einem Abscheider vom Katalysator abgetrennt.

Die Katalysatorabtrennung erfolgt zweckmäßigerweise im Temperaturbereich von 20 bis 200°C, bevorzugt von 40 bis 150°C und besonders bevorzugt von 40 bis 120°C unter einem Druck von 0,1 bis 60 bar, bevorzugt zwischen 1 und 10 bar und besonders bevorzugt zwischen 1 und 5 bar, wobei der Druck zweckmäßigerweise durch CO oder einem Inertgas und besonders bevorzugt von dem kohlenmonoxidhaltigen Reaktorabgas erzeugt wird. Der Sauerstoffpartialdruck in der Gasphase über der Reaktionsmischung beträgt hierbei höchstens 5 Vol.-%, bevorzugt höchstens 1 Vol.-% und besonders bevorzugt höchstens 0,5 Vol.-%. Überraschenderweise wird der Katalysator bei dieser Fahrweise des Reaktors und/oder des Abscheiders in vorzüglich sedimentierbarer Form erhalten und gleichzeitig eine klare, wasserhelle und katalysatorfreie Reaktionslösung erhalten. In der abgetrennten Reaktionslösung konnten weder Cu noch Anionen des Katalysators nachgewiesen werden. Die verbliebene Katalysatorsuspension, die aus Kupfer(I)-chlorid und etwas Reaktionslösung besteht, kann ohne weitere Aufarbeitung wieder eingesetzt werden. Dies war ein nicht vorhersehbarer Effekt, da nach dem Stand der Technik die Abtrennung des Katalysators von der Reaktionslösung als großes Problem gilt, und die nachgeschalteten Apparate hierdurch ebenfalls korrosionsfest ausgelegt werden müssen.

Das vom Katalysator befreite Reaktionsgemisch wird in grundsätzlich bekannter Weise durch Rektifikation aufgetrennt.

In einer vorteilhaften Variante wird im besonders bevorzugten Fall, daß R Methyl bedeutet, die katalysatorfreie Reaktionslösung durch einfache Rektifikation vom Reaktionswasser befreit. Diese Rektifikation wird bei 0,1 bis 15 bar, bevorzugt 0,8 bis 10 bar, besonders bevorzugt bei 1 bis 5 bar durchgeführt. Der Kolonnensumpf wird hierbei auf 65 bis 200°C, bevorzugt 80 bis 160°C, besonders bevorzugt auf 90 bis 150°C geheizt. Hierbei wird Wasser als Sumpfprodukt erhalten, während das Dimethylcarbonat und überschüssiges Methanol als Kopfprodukte erhalten werdend. In überraschender Weise und entgegen der einschlägigen Literatur wird hierbei keines der Azeotrope Dimethylcarbonat/Methanol/Wasser beziehungsweise Dimethylcarbonat/Wasser erhalten, sondern eine Trennung zwischen Dimethylcarbonat und Methanol einerseits und Reaktionswasser andererseits erreicht. Aufgrund der erfindungsgemäß angestrebten Zusammensetzung der Reaktionslösung mit überschüssigem Methanol wird bei einer solchen Destillation zunächst das Azeotrop Dimethylcarbonat/Methanol und danach weiteres überschüssiges Methanol am Kopf der Kolonne erhalten. In einer bevorzugten Ausführungsform dieser destillativen Trennung wird das Azeotrop Dimethylcarbonat/Methanol als Kopfprodukt, das weitere überschüssige Methanol als Seitenstrom und das Reaktionswasser als Sumpfprodukt erhalten. Die Trennung des Azeotrops Dimethylcarbonat/Methanol kann anschließend in grundsätzlich bekannter Weise, beispielsweise in einer unter höherem Druck betriebenen Kolonne, vorgenommen werden.

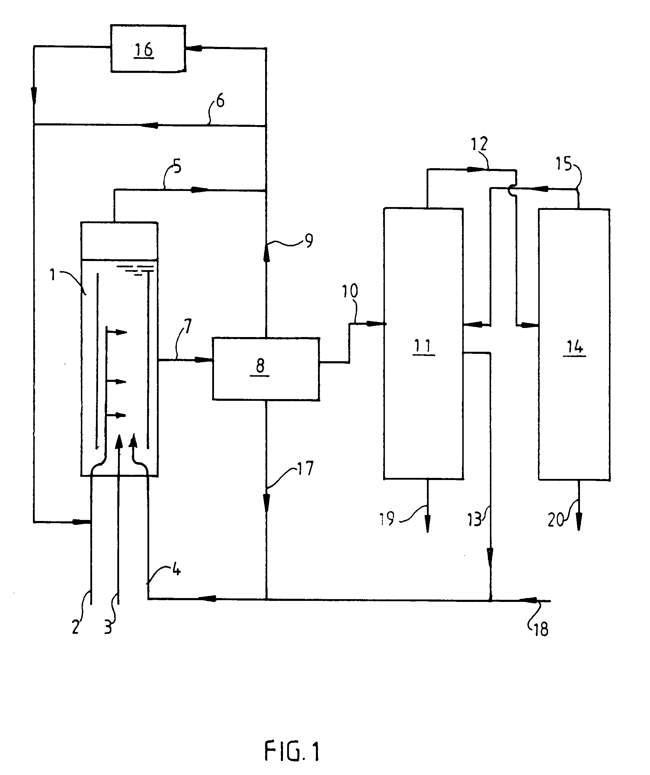

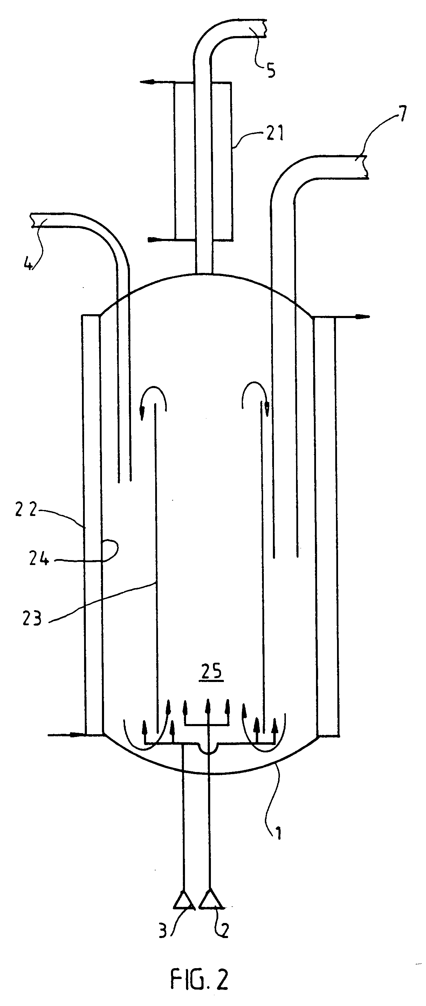

Anhand von Figur 1 soll im folgenden das erfindungsgemäße Verfahren am Beispiel der besonders bevorzugten Herstellung von Dimethylcarbonat beschrieben werden:
Die Umsetzung der katalysatorhaltigen Reaktionsmischung aus Methanol, Sauerstoff und Kohlenmonoxid erfolgt in einem druckfesten und korrosionsbeständigen Reaktor (1). Korrosionsbeständige Reaktormaterialien beziehungsweise Auskleidungsmaterialien sind beispielsweise Industriekeramik, Email, Teflon, Tantal, Inconel und andere dem Fachmann bekannte Materialien. Als Druckreaktoren sind grundsätzlich auch Autoklaven mit Rühreinrichtungen geeignet (DE-C 2 743 690, EP 365 083 A1). Wegen der guten Gasverteilung sind aber auch Blasensäulenreaktoren und insbesondere Schlaufenreaktoren mit internem Kreislauf geeignet. Wegen der schlanken Bauweise sind solche Schlaufenreaktoren insbesondere für mittlere und hohe Drücke geeignet. Sie zeichnen sich ferner durch die gute Dispergierung von Gasen und Feststoffen und das Fehlen beweglicher Teile aus (Chem.-Ing.-Tech. 52 (1980), 910/911; Chem.-Ing.-Tech. 62 (1990), 945-947). Es ist erfindungsgemäß möglich, sowohl in Einzelreaktoren als auch Reaktorkaskaden zu arbeiten. Über die Leitung (2) wird Kohlenmonoxid und über die Leitung (3) Sauerstoff in den Reaktor eingeleitet. Die Dispergiervorrichtungen für die Gase in vorhandene Suspension hinein sind bekannt und stellen beispielsweise Düsen dar. Es hat sich als vorteilhaft erwiesen, Kohlenmonoxid und Sauerstoff getrennt in den Reaktor einzuführen. Dies erlaubt beispielsweise die Dosierung des Kohlenmonoxids in vorteilhafter Weise simultan in unterschiedlichen Höhen in den Reaktor hinein (Abzweigungen von (2)). Über die Leitung (4) wird dem Reaktor (1) Methanol, bestehend aus Frisch-Methanol und Rückführungs-Methanol, und der darin suspendierte kupferhaltige Katalysator zugeführt. In bevorzugter Weise erfolgt auch die Zuführung des Methanols mit dem Katalysator in den unteren Reaktorbe reich oder zwischen Außen- und Innenrohr, wie in Figur 2 beschrieben. Das Reaktorabgas verläßt über die Leitung (5) den Reaktor (1) und wird über die Kreislaufleitung (6) der Leitung (2) zur erneuten Ausnutzung von noch vorhandenen Kohlenmonoxid und Sauerstoff zugeführt. Für den Fall, daß etwa mitentstandenes CO₂ aus dem Kreislaufgas entfernt werden soll, kann ein Teil dieses Kreislaufgases durch eine CO₂-Wäsche (16) geleitet und dann wieder zurückgeführt werden. Für den Fall, daß für das Kohlenmonoxid oder den Sauerstoff eine Verdünnung mit Inertgasen vorgesehen ist, muß selbstverständlich für eine ausreichende Ausschleusung von Abgas gesorgt werden.

Über die Leitung (7) wird kontinuierlich katalysatorhaltiges Reaktionsgemisch dem Reaktor (1) entnommen, dem Abscheider (8) zugeführt und entspannt. Der Abscheiderdruck wird zweckmäßigerweise durch das entspannte Reaktionsabgas eingestellt. Das Abscheiderabgas wird über die Leitung (9) der Kreisgasleitung (6) in komprimierter Form wieder zugeführt.

Die Abtrennung des Katalysators kann durch Sedimentation und/oder Zentrifugieren, beispielsweise in einem Hydrozyklon beziehungsweise Hydrozentriklonen erfolgen. Als Sedimentationsbehälter eignen sich beispielsweise Einkammer- oder Mehrkammereindicker (Dorreindicker), Klassierer, Spitzkästen, Lamellenabscheider, Röhrensedimentationsanlagen und weitere dafür entwickelte Apparate (Chem.-Ing.-Tech. 52 (1980), 332; Kirk-Othmer, Encyclopedia of Chemical Technology, Vol. 20, Third Edition, John Wiley & Sons, New York 1982). Als Zentrifugen sind geeignet: Dekanter, Tellerzentrifugen, Überlauf- und Schälrohrzentrifugen und andere. Die eingedickte Katalysatorsuspension wird über die Leitung (17) gemeinsam mit Frisch-Methanol (18) und Rück-Methanol (13) über die Leitung (4) dem Reaktor (1) wieder zugeführt.

Aus dem Abscheider (8) fließt über die Leitung (10) eine klare, katalysatorfreie Reaktionslösung, bestehend aus Dimethylcarbonat, überschüssigem Methanol und Reaktionswasser, in die Destillationskolonne (11). In (11) wird eine Trennung in das Reaktionswasser als Sumpfprodukt (19), in Methanol, das in einem Seitenstrom (13) abgenommen und in die Reaktion zurückgeführt wird, und in das Dimethylcarbonat/Methanol-Azeotrop als Kopfprodukt vorgenommen. Als Kolonnen sind beispielsweise Füllkörperkolonnen, Glockenbodenkolonnen und andere geeignet. Das als Kopfprodukt abgehende Dimethylcarbonat/Methanol-Azeotrop wird über die Leitung (12) der Dimethylcarbonat-Kolonne (14) zugeleitet. In (14) erfolgt eine an sich bekannte Auftrennung des Dimethylcarbonat/Methanol-Azeotrops, beispielsweise durch Destillation unter erhöhtem Druck. Als Sumpfprodukt (20) von (14) wird Dimethylcarbonat abgezogen. Als Kopfprodukt (15) von (14) fällt ein an Dimethylcarbonat verarmtes Methanol an, welches über die Leitung (15) der Destillationskolonne (11) wieder zugeführt wird. Das auf diese Weise gewonnene Dimethylcarbonat (20) ist von hoher Reinheit und kann ohne weitere Reinigung vielen vorgesehenen Verwendungszwecken zugeführt werden.

In Figur 2 ist der erfindungsgemäß bevorzugt zu verwendende Reaktor schematisch dargestellt.

Dem Reaktor (1), bestehend aus einem Innenrohr (Zentralrohr) (23) oder mehreren Innenrohren und einem Außenrohr (24) mit Wärmeaustauschfläche (22) wird über die Leitung (2) Kohlenmonoxid in das oder die Innenrohr(e) zugeführt. Die Sauerstoffdosierung erfolgt getrennt über Leitung (3) ringförmig in Innen- und/oder Außenrohr. Die Gasverteilung (25) erfolgt nach dem Stand der Technik z.B. durch Düsen, Schlitze, Fritten, Siebe usw. Aufgrund der in das Innenrohr dosierten Gasmenge von 500-30.000 Nl/l Flüssigkeitsvolumen, bevorzugt 800-20.000 Nl/l Flüssigkeitsvolumen und besonders bevorzugt 1000-15.000 Nl/l Flüssigkeitsvolumen, wird die Suspension nach dem Prinzip der Mammutpumpe gefördert. Über Leitung (4) erfolgt die Zuführung von Frisch-Methanol, Rückführungs-Methanol und recyclisiertem und evtl. frischem Katalysator. Gleichzeitig wird über Leitung 7, die einen vergleichsweise großen Querschnitt besitzt, eine entsprechende Menge Reaktionslösung und Katalysator entnommen. Aufgrund der Dimensionierung und der Entnahme entgegen der Schwerkraft wird bereits ein großer Teil des Katalysators von der Reaktionslösung abgetrennt. Der restliche Katalysator wird wie beschrieben durch Sedimentation abgetrennt.

Über Leitung (5) verläßt nicht abreagiertes Reaktionsgas den Reaktor, wobei mitgerissene Flüssigkeit und Dampf am Kühler (21) kondensiert werden. Die Temperierung des Reaktors erfolgt über die Wärmeaustauschfläche (22).

Der erfindungsgemäß bevorzugt zu verwendende Schlaufenreaktor mit internem Stoffkreislauf (interner Schlaufe) ist in der bevorzugten Ausführungsform ein schlanker Reaktor mit 0,1 bis 40 m, bevorzugt 0,5 - 30 m und besonders bevorzugt 1 bis 20 m Höhe, bei einem Durchmesser des äußeren Rohres von 0,1-5 m, bevorzugt 0,3-4 m und besonders bevorzugt von 0,5-2 m. Das Verhältnis der Volumina des oder der Innenrohre, zum äußeren Stoffkreislaufvolumen beträgt 0,1-5, bevorzugt 0,2 bis 3 und besonders bevorzugt 0,5 bis 2.

### Beispiel 1

In einem 2 l-Tantalautoklaven mit Rührer wurden 1000 ml Methanol bei 30 bar und 120°C mit Sauerstoff und Kohlenmonoxid umgesetzt. Als Katalysator wurde Cu₂Cl₂ in einer Menge von 1,62 Mol/Liter Methanol verwendet. Die Mengendurchflüsse von Kohlenmonoxid und Sauerstoff betrugen 120 beziehungsweise 6,3 NL/h. Die Reaktionsmasse wurde durch das Rührorgan und die Strömung der Reaktionsgase in Bewegung gehalten. Nach 4 h Reaktionszeit wurde der Autoklav unter Schutzgas entleert.

Der Katalysator wurde unter CO-Gas (1 bar) durch Sedimentation und Dekantieren bei 40°C von der klaren und wasserhellen Reaktionslösung abgetrennt. Die Analyse der Reaktionslösung ergab folgende Zusammensetzung (in Gew.-%): 75 % MeOH, 20,6 % DMC und 4,4 % H₂O. Kupfer- und Chloridionen konnten nicht nachgewiesen werden. Das Abgas des Autoklaven bestand lediglich aus CO (95-99 %), CO₂ (0,04-1 %) und O₂ (0-0,8 %). Methylchlorid und Dimethylether konnten nur in geringsten Mengen nachgewiesen werden (0,006-0,03 Vol.-% in mehreren Wiederholungsversuchen). Die Analyse des weißen Katalysators ergab als Zusammensetzung Cu₂Cl₂ mit etwas Wasser.

### Beispiel 2

Die Umsetzung erfolgte analog zum Beispiel 1, jedoch mit dem Unterschied, daß statt des Kupfer(I)chlorids Kupfermethoxychlorid als Katalysator verwendet wurde, welches durch Voroxidation des eingesetzten CuCl (1,62 Mol/L MeOH) bei 120°C und 20 bar Stickstoffvordruck mit Sauerstoff (1,1 Mol) im Autoklav hergestellt worden war.

Danach erfolgte die Aufarbeitung analog zum Beispiel 1.

Die Analyse der klaren, wasserhellen und katalysatorfreien Reaktionslösung ergab folgende Zusammensetzung: 68 % MeOH, 26 % DMC und 6,0 % Wasser. Die Selektivität betrug, bezogen auf CO, für DMC 93,1 und 6,9 % für CO₂.

Die Analyse des weißen Katalysators ergab: 63,0 % Cu, 33,7 % Cl, < 0,1 % C, 0,3 % H und 2,9 % O₂.

Eine während der Reaktion aus dem Reaktor entnommene Suspensionsprobe (23 g) sedimentierte unter Luftausschluß (Spülung mit CO) bei 40°C sofort. Eine andere entnommene Suspensionsprobe (39 g), die unter üblichen Bedingungen (keine CO-Spülung) bei Luft-Kontakt sedimentieren sollte, konnte weder sedimentiert noch filtriert werden. Eine Abtrennung des Katalysators gelang nur durch Abdestillieren der Reaktionslösung.

### Beispiel 3

In dem im Beispiel 1 beschriebenen Autoklaven wurde Methanol bei 30 bar und 120°C mit Sauerstoff und Kohlenmonoxid analog zum Beispiel 2 umgesetzt. Im Gegensatz zum Beispiel 2 wurde der Autoklav nach Reaktionsende nicht geöffnet, sondern nach der Abkühlung und Sedimentation des Katalysators (T=50°C) wurde die Reaktionslösung über ein Steigrohr aus dem Autoklaven entfernt.

Nach der Zugabe von frischem Methanol erfolgte die Umsetzung analog zum Beispiel 1.

Dieser Vorgang wurde insgesamt viermal durchgeführt und beim 5. Zyklus der Autoklav analog zum Beispiel 1 entleert.

In Tabelle 1 sind die Ergebnisse des Versuchs zusammengestellt. In allen Fällen wurden klare und wasserhelle Reaktionslösungen erhalten. Auch hier lagen der Cu- und Cl-Gehalt in den Reaktionslösungen unter der Nachweisgrenze. Die Abgaszusammensetzungen entsprachen der des Beispiels 2.

**Tabelle 1**

| Nr. | Einzellaufzeit h | DMC-Gehalt der Reaktionslösung Gew.-% | Selektivitäten | | |
|---|---|---|---|---|---|
| | | | bezogen auf MeOH an DMC | bezogen auf CO | |
| | | | | an DMC | an CO₂ |
| 1 | 2 | 18,3 | >99 | 90,8 | 9,2 |
| 2 | 4 | 22,2 | >99 | 92,4 | 7,6 |
| 3 | 4 | 27,7 | >99 | 93,4 | 6,6 |
| 4 | 5 | 28,5 | >99 | 92,6 | 7,4 |
| 5 | 5 | 28,9 | >99 | 90,9 | 9,1 |
| DMC = Dimethylcarbonat; MeOH = Methanol | | | | | |

### Beispiel 4

In einer Destillationsapparatur, bestehend aus einem Zweihalskolben, 15 cm-Füllkörperkolonne mit 8 cm Füllhöhe (4 mm Berl-Sättel), Claisenaufsatz und Fraktionssammler wurden bei Normaldruck 125 g katalysatorfreie Reaktionslösung aus Beispiel 4 destilliert. Die Reaktionslösung hatte folgende Zusammensetzung: 70,9 % MeOH, 23,8 % DMC, 5,3 % H₂O. Schon mit dieser einfachen Anordnung wurde folgendes Trennergebnis erzielt (Tabelle 2):

**Tabelle 2**

| Fraktion | Temperatur | | Menge g | Zusammensetzung in Gew.-% | | |
|---|---|---|---|---|---|---|
| | Sumpf | Kopf | | MeOH | DMC | H₂O |
| 1 | 100 | 64 | 105,33 | 71,1 | 28,6 | 0,3 |
| 2 | 100 | 64 | 8,79 | 98,2 | 0,7 | 1,0 |
| Rest | - | - | 10,70 | 42,4 | 0,0 | 57,6 |

### Beispiel 5

In einer Destillationsapparatur, bestehend aus einem Dreihalskolben, einer 100 cm langen Kolonne, gefüllt mit 4x4 mm V₂A-Maschendrahtröllchen und Fraktionssammler wurde eine katalysatorfreie Lösung folgender Zusammensetzung destilliert; 71,4 % MeOH, 23,8 % DMC und 4,8 % Wasser.

Aus dem Sumpf wurden während des Versuchs 64,6 g Lösung zwecks Analysen entnommen. Bei der Destillation wurden folgendes Ergebnis erzielt:

**Tabelle 3**

| Fraktion | Temperatur | | Menge g | Zusammensetzung in Gew.-% | | |
|---|---|---|---|---|---|---|
| | Sumpf | Kopf | | MeOH | DMC | H₂O |
| 1 | 107 | 64,1 | 382,7 | 70,7 | 29,3 | < 0,1 |
| 2 | 107 | 64,7 | 69,6 | 92,0 | 8,0 | < 0,1 |
| Rest | - | - | 27,9 | 45,7 | 0,0 | 54,3 |

## Patentansprüche

1. Verfahren zur Herstellung von Dialkylcarbonaten der Formel
RO-CO-OR,
in der
R für C₁-C₁₀-Alkyl, bevorzugt für C₁-C₂-Alkyl, besonders bevorzugt für CH₃ steht,
durch Umsetzung des zugrunde liegenden Alkanols ROH mit Sauerstoff und Kohlenmonoxid in Gegenwart eines Kupferkatalysators bei erhöhter Temperatur und erhöhtem Druck, dadurch gekennzeichnet, daß der Katalysator in unlöslicher Form durch mechanische Abtrennung bei 20 bis 200°C, einem Druck von 0,1-60 bar und einem Sauerstoff-Partialdruck über dem Reaktionsgemisch von höchstens 5 Vol.-% abgetrennt wird und das vom Katalysator befreite Reaktionsgemisch durch Rektifikation aufgearbeitet wird.

2. Verfahren nach Anspruch 1, dadurch gekennzeichnet, daß Cu(I)- und/oder Cu(II)-Verbindungen aus der Gruppe der anorganischen und organischen Cu-Salze und der Alkoxysalze von Cu als Katalysator eingespeist werden.

3. Verfahren nach Anspruch 1, dadurch gekennzeichnet, daß als mechanische Abtrennung Abdekantieren oder Abhebern nach Sedimentation, Filtration oder Zentrifugieren durchgeführt wird.

4. Verfahren nach Anspruch 1, dadurch gekennzeichnet, daß die Reaktion bei 60 bis 200°C und bei 1 bis 60 bar durchgeführt wird.

5. Verfahren nach Anspruch 1, dadurch gekennzeichnet, daß das dem Reaktor entnommene Reaktionsgemisch die molare Zusammensetzung Alkanol:Dialkylcarbonat:Cu = 1:0,005-1:0,0001-5 hat.

6. Verfahren nach Anspruch 1, dadurch gekennzeichnet, daß Kohlenmonoxid und Sauerstoff im molaren Verhältnis CO:O₂ = 1:0,005-1 eingesetzt werden.

7. Verfahren nach Anspruch 1, dadurch gekennzeichnet, daß eine Konzentration des Reaktionswassers im Reaktionsgemisch von höchstens 8 Gew.-%, bezogen auf das Gewicht des flüssigen Reaktionsgemisches, eingestellt wird,

8. Verfahren nach Anspruch 1, dadurch gekennzeichnet, daß im Falle von R = CH₃ das Reaktionswasser bei der Rektifikation bei 0,1-15 bar und einer Sumpftemperatur von 65-200°C als Sumpfprodukt ausgeschleust wird,

9. Verfahren nach Anspruch 1, dadurch gekennzeichnet, daß die Umsetzung bis zu einem Wert von kleiner als 35 % des eingesetzten Alkanols getrieben wird.

10. Verfahren nach Anspruch 1, dadurch gekennzeichnet, daß ein Schlaufenreaktor mit internem Stoffkreislauf mit 0,1-40 m Höhe bei einem Durchmesser des äußeren Rohres von 0,1-5 m und einem Verhaltnis der Volumina des oder der Innenrohre zum äußeren Stoffkreislaufvolumen von 0,1-5 verwendet wird.

## Claims

1. Process for the preparation of dialkyl carbonates of the formula
RO-CO-OR
in which
R represents C₁-C₁₀-alkyl, preferably C₁-C₂-alkyl, particularly preferably CH₃,
by reacting the starting alkanol ROH with oxygen and carbon monoxide in the presence of a copper catalyst at elevated temperature and elevated pressure, characterised in that the catalyst in insoluble form is separated off by mechanical separation at 20 to 200°C, at a pressure of 0.1-60 bar and at an oxygen partial pressure above the reaction mixture of at most 5 % by volume and the reaction mixture freed from the catalyst is worked up by rectification.

2. Process according to Claim 1, characterised in that Cu(I) compounds and/or Cu(II) compounds selected from the group consisting of the inorganic and organic Cu salts and alkoxy salts of Cu are fed in as catalyst.

3. Process according to Claim 1, characterised in that the mechanical separation carried out is decanting or siphoning after sedimentation, filtration or centrifuging.

4. Process according to Claim 1, characterised in that the reaction is carried out at 60 to 200°C and at 1 to 60 bar.

5. Process according to Claim 1, characterised in that the reaction mixture withdrawn from the reactor has the molar composition alkanol:dialkyl carbonate: Cu = 1:0.005-1:0.0001-5.

6. Process according to Claim 1, characterised in that carbon monoxide and oxygen are used in the molar ratio CO:O₂ = 1:0.005-1.

7. Process according to Claim 1, characterised in that a concentration of the reaction water in the reaction mixture is established of at most 8 % by weight, relative to the weight of the liquid reaction mixture.

8. Process according to Claim 1, characterised in that in the case of R = CH₃, the reaction water is discharged as bottom product in the rectification at 0.1-15 bar and a bottom temperature of 65-200°C.

9. Process according to Claim 1, characterised in that the reaction proceeds to a value of less than 35 % of the alkanol used.

10. Process according to Claim 1, characterised in that a loop reactor having internal material circulation is used of height 0.1-40 m at a diameter of the outer tube of 0.1-5 m and a ratio of the volumes of the inner tube or tubes to the outer material circulation volume of 0.1-5.

## Revendications

1. Procédé de préparation de carbonates de dialkyle de formule
RO-CO-OR,
dans laquelle
R représente un groupe alkyle en C₁-C₁₀, de préférence en C₁-C₂ et tout particulièrement CH₃,
par réaction de l'alcanol correspondant ROH avec l'oxygène et le monoxyde de carbone en présence d'un catalyseur au cuivre, à température et pression élevées, caractérisé en ce que l'on sépare le catalyseur à l'état insoluble par séparation mécanique, à des températures de 20 à 200°C, sous une pression de 0,1 à 60 bar et avec une pression partielle d'oxygène sur le mélange de réaction qui est au maximum de 5 % en volume, puis on traite par rectification le mélange de réaction débarrassé du catalyseur.

2. Procédé selon la revendication 1, caractérisé en ce que l'on utilise en tant que catalyseurs des composés cuivreux et/ou cuivriques choisis parmi les sels minéraux et organiques du cuivre et les alcoxysels du cuivre.

3. Procédé selon la revendication 1, caractérisé en ce que la séparation mécanique est réalisée par décantation ou pompage du liquide surnageant après sédimentation, filtration ou centrifugation.

4. Procédé selon la revendication 1, caractérisé en ce que la réaction est réalisée à des températures de 60 à 200°C et des pressions de 1 à 60 bar.

5. Procédé selon la revendication 1, caractérisé en ce que le mélange de réaction évacué du réacteur est à la composition molaire alcanol/carbonate de dialkyle/Cu de 1 : 0,005 à 1 : 0,0001 à 5.

6. Procédé selon la revendication 1, caractérisé en ce que l'on met en oeuvre le monoxyde de carbone et l'oxygène à un rapport molaire CO/O₂ de 1 : 0,005 à 1.

7. Procédé selon la revendication 1, caractérisé en ce que l'on règle la concentration de l'eau de réaction dans le mélange de réaction à 8 % en poids au maximum par rapport au poids du mélange de réaction liquide.

8. Procédé selon la revendication 1, caractérisé en ce que, lorsque R = CH₃, on élimine l'eau de réaction par rectification sous une pression de 0,1 à 15 bar à une température de pied de colonne de 65 à 200°C, en tant que produit de pied de colonne.

9. Procédé selon la revendication 1, caractérisé en ce que la réaction est poursuivie jusqu'à un taux de conversion inférieur à 35 % pour l'alcanol mis en oeuvre.

10. Procédé selon la revendication 1, caractérisé en ce que l'on utilise un réacteur à circuit avec circuit interne de matières, d'une hauteur de 0,1 à 40 m, avec un diamètre du tube extérieur de 0,1 à 5 m et un rapport de 0,1 à 5 entre le volume du ou des tubes intérieurs et le volume extérieur du circuit de matières.
